# EUROPEAN PATENT APPLICATION

(11) **EP 3 453 419 A1**
(43) Date of publication of application: **13.03.2019**
(21) Application number: 17306171.4
(22) Date of filing: 12.09.2017
(51) Int. Cl.: A61M 21/02, A47C 21/04, A61F 7/08, A61B 5/01, A61B 5/00, A61M 21/00, A61F 7/00, A61F 7/02, A61B 5/11

(54) **METHOD AND SLEEP DEVICE FOR SLEEP REGULATION**

(71) Applicant: Moona, 75010 Paris (FR)
(72) Inventor: JUIN, Coline, 75009 Paris (FR); STOIKOVITCH, David, 92400 Courbevoie (FR)
(74) Representative: Icosa

(57) **Abstract**

The present invention relates to a method for regulating the temperature of a sleep device, comprising at least one sensor, the method comprising the following steps: defining (DEF) a predefined user data (DATA1), receiving (RCV) dynamic user data (DATA2), said dynamic user data comprising data collected from a user interaction with at least one sensor of the sleep device, calculating (K) at least one sleep evaluation variable (VAR) evaluating the quality of sleep of the user using at least one part of the dynamic user data (DATA2) and/or predefined user data (DATA1), generating (GEN) a user temperature profile (PROF) using a profiling model (MODp) having as input the at least one sleep evaluation variable (VAR) and at least one part of the user data (DATA1, DATA2); where the user temperature profile (PROF) comprises a plurality of associations, each of said association comprising a temperature and a period of time of the user sleep cycle; and regulating (REG) the sleep device by generating a temperature instruction calculated with the user temperature profile (PROF). The invention also relates to a sleep device and system comprising said sleep device.

## Description

### FIELD OF INVENTION

The present invention pertains to the field of sleep regulation technologies. In particular, the devices and systems described herein generally relate to apparatuses and methods for improving sleep (including reducing sleep onset and maintenance of sleep duration), enhancing or increasing sleep, including, but not limited to, improving ease of awakening, treating sleeping disorders such as insomnia.

### BACKGROUND OF INVENTION

Although sleep disruption and irregularities, including insomnia, are a widespread problem, a good quality of sleep is essential to one's physical health and emotional well-being. Sleep loss may influence someone's mood, physical and mental performances, efficiency, and ability to handle stress.

The sleep process comprises phases that are governed by the circadian rhythm. The circadian rhythm is a biological process presenting an endogenous entrainable oscillation occurring within the body every 24-hour period. The circadian rhythm is a fundamental property possessed by all organisms and these rhythms are driven by an internal timekeeping system.

One circadian related physiological phenomenon in humans is the variation in body core temperature during the various sleep phases. In such a process, the body core temperature begins to drop after a person begins to fall asleep. The core temperature remains below normal throughout the night, but begins to increase to normal levels sometime before wakening.

It is known from literature that controlled regulation body temperature variation across the 24-hour cycle influences the sleep cycle of a subject.

The US patent application US 2015/0283353 describes a sleep system, comprising a thermal pad to be put in proximity of a subject, configured to automatically adjust the temperature of the thermal pad during the sleep cycle time according to a programmed routine. The different temperatures of the programmed routine are pre-set temperatures or subject selected. In the first case, the temperature variation applied during the sleep cycle does not take in consideration the sleeping habits of the subject and his specificities, while in the second case, the pertinence and efficiency of the sleep device depends on the subject choices and therefore on his experience and auto-evaluation abilities.

Since the regulation of body temperature directly influences the circadian rhythm, a finer and subject adapted delineation of temperature profile to be delivered to subject during the night would be preferable.

It is therefore an object of the present invention to provide a method to obtain an optimal subject dedicated temperature profile to be delivered to the subject during the night time in order to improve the subject quality of sleep.

### SUMMARY

The present invention relates to a method for regulating the temperature of a sleep device, comprising at least one sensor, the method comprising the following steps:
a) defining a predefined user data;
b) receiving dynamic user data, said dynamic user data comprising data collected from a user interaction with at least one sensor of the sleep device;
c) calculating at least one sleep evaluation variable evaluating the quality of sleep of the user using at least one part of the dynamic user data and/or predefined user data;
d) generating a user temperature profile using a profiling model having as input the at least one sleep evaluation variable and at least one part of the user data; where the user temperature profile comprises a plurality of associations, each of said association comprising a temperature and a period of time of the user sleep cycle; and
e) regulating the sleep device by generating a temperature instruction calculated with the user temperature profile.

According to one embodiment, the at least one sleep evaluation variable is calculated using a scoring model and at least one part of the dynamic user data and/or predefined user data.

According to one embodiment, the dynamic user data comprises a previous user temperature profile generated by a previous use of the method.

According to one embodiment, the predefined user data comprises information obtained from a sleep assessment survey.

According to one embodiment, the method of the present invention further comprises a step of preprocessing of the dynamic user data received from the at least one sensor of the sleep device.

According to one embodiment, the profiling model is configured to further receive as input a first timestamp corresponding to the begin of the sleep cycle and a second timestamp corresponding to the end of the sleep cycle.

According to one embodiment, a first timestamp corresponding to the begin of the sleep cycle and a second timestamp corresponding to the end of the sleep cycle are derived from the processing of the dynamic user data.

According to one embodiment, the method of the present invention further comprises the step of transmitting the temperature instruction to the sleep device.

The present invention further relates to a sleep device using body temperature conditioning, comprising:
a) a thermal pad;
b) at least one sensor;
c) a communication means configured to transmit the information detected by the at least one sensor and receive the user temperature profile generated according to the method described hereabove; and
d) a thermal unit adjusting a temperature of the thermal pad during a sleep cycle of the user according to the user temperature profile generated.

According to one embodiment, the at least one sensor of the sleep device is at least one of the following: an ambient temperature sensor, a movement sensor, a humidity sensor, a sound sensor or/and a light sensor.

The present invention further relates to a system comprising a sleep device including at least one sensor, a user interface for user interaction and a thermal control unit, executing the temperature instruction obtained from the method according to anyone of the embodiment described hereabove.

### DEFINITION

In the present invention, the following term has the following meaning:
- "**Sleep cycle**" refers to the period of time during which a subject sleep during the night, with or without interruption.

### DETAILED DESCRIPTION

The following detailed description will be better understood when read in conjunction with the drawings. For the purpose of illustrating, the device is shown in the preferred embodiments. It should be understood, however that the application is not limited to the precise arrangements, structures, features, embodiments, and aspect shown. The drawings are not drawn to scale and are not intended to limit the scope of the claims to the embodiments depicted. Accordingly, it should be understood that where features mentioned in the appended claims are followed by reference signs, such signs are included solely for the purpose of enhancing the intelligibility of the claims and are in no way limiting on the scope of the claims.

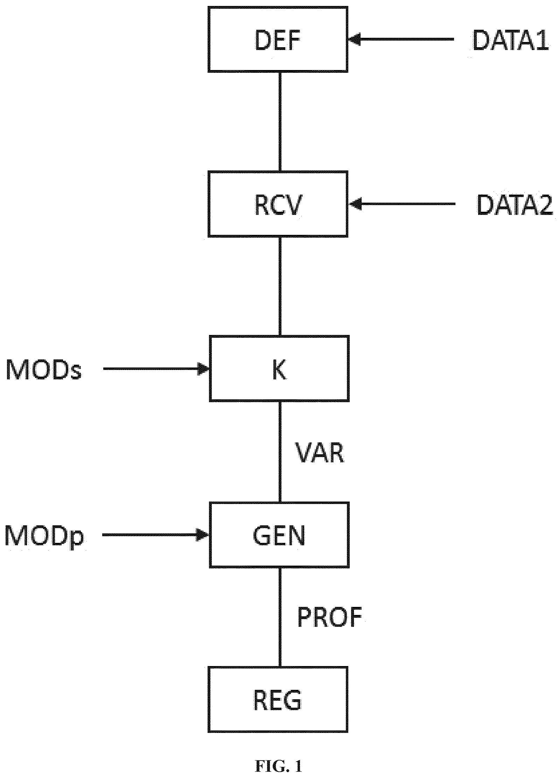

According to one aspect, the present invention concerns a method for regulating the temperature of a sleep device, comprising at least one sensor. As shown in Figure **1****,** the method comprises the following main steps:
a) defining **DEF** a predefined user data **DATA1;**
b) receiving **RCV** dynamic user data **DATA2;**
c) calculating **K** at least one sleep evaluation variable **VAR** evaluating the quality of sleep of the user;
d) generating **GEN** a user temperature profile **PROF** using a profiling model **MODp**; and
e) regulating **REG** the sleep device by generating a temperature instruction calculated with the user temperature profile **PROF.**

### DEVICE

In another aspect, the invention relates to a sleep device. In order to better understand the method of the present invention, the sleeping device will be described first.

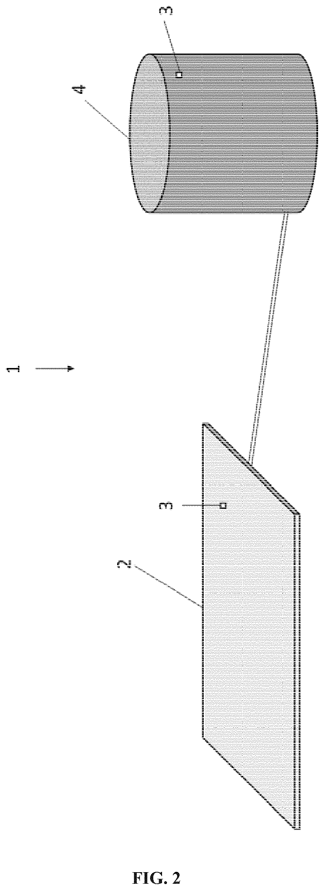

With reference to Figure **2****,** the sleep device **1,** according to the present invention comprises at least one thermal pad **2,** at least one sensor **3,** at least one thermal unit and a communication means. According to one embodiment, the sleep device comprises a thermal unit configured to adjust a temperature of the thermal pad during a sleep cycle of the user according to the user temperature profile generated from the method of the present invention.

According to one embodiment, the thermal unit and the communication means are both enclosed in a housing **4.**

According to one embodiment, the device is configured with the following embodiments of the method described below.

According to one embodiment, the thermal unit employs at least one of the following thermal transfer fluid to modify the temperature of thermal pad **2**: direct heat, warm gas, warm liquid, a warming gel, a cooling gel, a cool liquid and/or a cool gas.

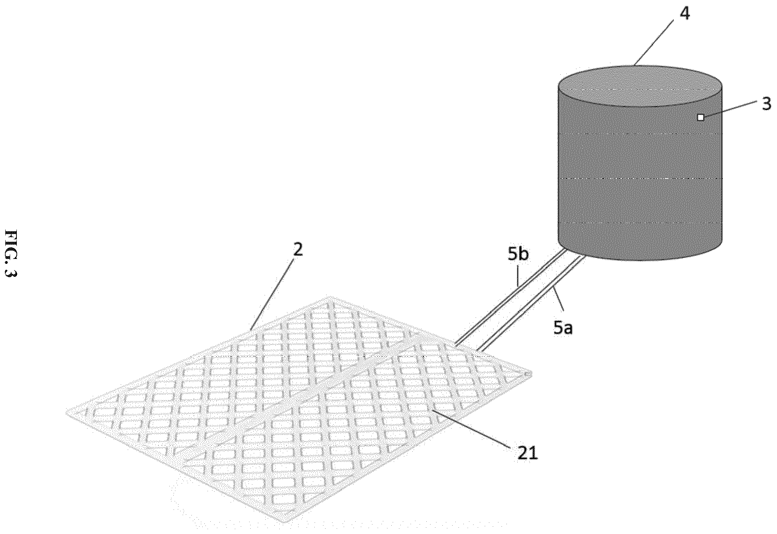

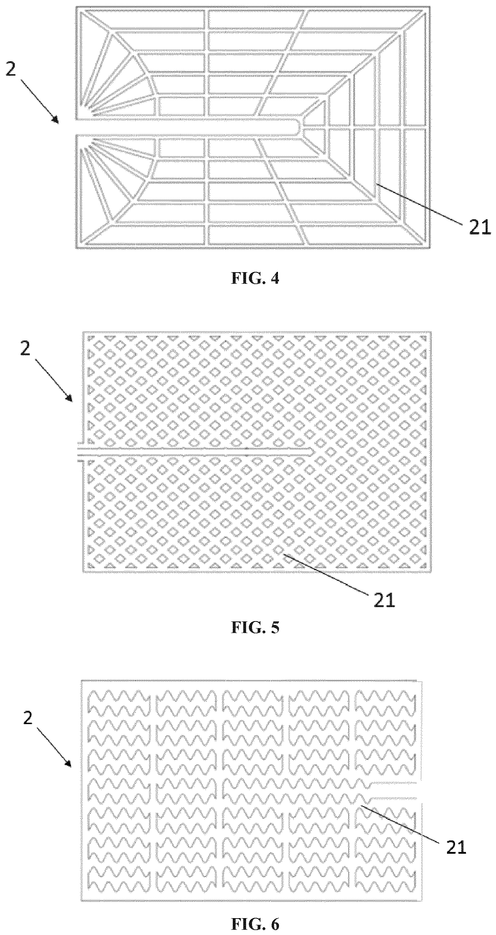

According to a preferred embodiment, the device further comprises a fluidic circuit configured to put in fluidic communication the thermal unit and the thermal pad **2.** Thermal transfer fluids such as air, water, vapor and/or gas(es) may be distributed throughout at least one portion of the thermal pad **2** via at least oneone perforated tubing. The at least one perforated tubing may be flexible, semi-rigid, rigid, combinations thereof, and/or the like. According to one embodiment, at least one of the materials of which the tubing is made of is non-permeable. The tubing may, for example, comprise at least one defined exit for accepting air, water vapor and/or gas(es). At least part of the at least one defined exit may, for example, be configured to direct air, water vapor and/or gas(es) through an outer surface of the thermal pas **2.** The at least one defined exit may be configured to direct air, water vapor and/or gas(es) towards and/or away from an intended user. The air, water vapor, and/or gas(es) may be accepted via at least one tube from a remote enclosure. The remote enclosure may be configured with air quality and movement devices to vary the moisture content, pressure, and/or flow rate of the air, water vapor, and/or gas(es). The air quality and movement devices may comprise without limitation: pumps, control electronics, filters, air dryers, combinations thereof, and/or the like. Thermal pad **2** may, for example, comprise a blow molded or twin sheet formed part comprising hollow conduits **21.** Said hollow conduits configure to allow circulation of a thermal transfer fluid. Sheets may be bonded together to form the thermal pad **2.** The hollow conduits may, for example, be arranged in a series of parallel runs in a planar fashion. Thermal pad **2** may further comprise input port to accept at least one thermal transfer fluid and output port configured to return at least one thermal transfer fluid, as show in Figure **3****.** The hollow conduits may, for example, be arranged in a grid with different crossing patterns in a planar fashion. Some examples of the patterns that may have the thermal pad **2** hollow conduits are reported in Figures **4** to **6****.**

According to one embodiment, the sleep device comprises an input transfer tube **5a** and an output tube transfer **5b** putting in fluidic communication the input and output of the thermal pad **2** with the thermal unit.

According to one embodiment, the thermal pad **2** is configured to be inserted in a mattress, a pillow or a blanket.

According to one embodiment, the thermal pad **2** comprises multiple regions fluidly isolated from each other, wherein each region comprises an input port to accept at least one thermal transfer fluid and output port configured to return at least one thermal transfer fluid. According to this embodiment, the multiple regions of the thermal pad **2** may be put at different temperatures; such configuration permits to transmit different temperatures to different body parts. According to this embodiment, the multiple regions of the thermal pad **2** may be heated or cooled at an equal temperature.

According to one embodiment where the thermal pad **2** is configured to be inserted in the pillow, the sleep device further comprises support means which matches the patterns of the empty space between the hollow conduits of the thermal pad **2** in order to comfortably support the head of the user and allow an easier circulation of the accepted at least one fluid, liquid, gel, air, and/or gas inside the hollow conduits. Said support means may be made of fabric, foam, plastic, silicon and/or the like. The support means may comprise at least one of the following material: fabric, foam, plastic, silicon and/or the like.

According to one embodiment, the at least one sensor **3** is at least one of the following sensors: an ambient temperature sensor, a movement sensor, an ambient humidity sensor, a sound sensor, an air quality and composition sensor and/or a light sensor.

The ambient temperature, the humidity sensor, the sound sensor, the movement sensor, light sensor or/and the air quality and composition sensor may be located on the housing **4** or anywhere else in the room where the subject is sleeping. The light sensor may be a light-dependent resistor or a digital light intensity meter. The sound sensor may be a microphone or a sound intensity meter, or any sound sensor known to those of skill in the art. The movement sensor, allowing detection of user movement during sleep, may be an accelerometer mechanically connected to the thermal pad **2** or alternatively a water pressure sensor or a water flow sensor in connection with the sleep device fluidic circuit.

According to one embodiment, the at least one sensor **3** is a body temperature thermometer, such as but not limited to infrared thermometer or a contact thermometer, placed on housing **4** or anywhere else in the subject bedroom or on the subject himself by means of supports know by the man skilled in the art.

The at least one sensor **3** may further be a heart rate sensor, such as but not limited to an electrocardiographic electrode, ultra wide band sensor, an optical sensor or a microwave sensor. The optimal positioning of these heart rate sensors is known by the man skilled in the art.

The at least one sensor **3** may further be a breathing sensor such as but not limited to ultra wide band sensor, nasal airflow sensor, or any other sensors known by the man skilled in the art to monitor a subject breathing. The optimal positioning of breathing sensors is known by the man skilled in the art.

The at least one sensor **3** may further be an electromyographic sensor. The optimal positioning of these sensors is known by the man skilled in the art.

The at least one sensor **3** may further be an electroencephalographic sensor. The optimal positioning of these sensors is known by the man skilled in the art.

According to one embodiment, the at least one sensor 3 wirelessly communicates with the communication means.

According to one embodiment, the communication means is configured to transmit the information detected by at least one sensor **3** and receive the user temperature profile **PROF** generated according to the method described hereabove.

The communication means may comprise at least one transceiver which may be, for example, connected to at least one processing unit. The transceiver may also, for example, be configured to communicate with at least one remote device. According one embodiments, the transceiver may employ at least one of the following communication mechanisms: a wired communications mechanism, a wireless communications mechanism, Ethernet, Wi-Fi, Bluetooth, ZigBee, a Wireless Personal Area Network (WPAN), Near Field Communications (NFC), an infrared connection, a combination thereof, and/or the like. The wired communications mechanism may comprise an external connector. The remote device may, for example, comprise a computing device, a server, a mobile device, a remote control, a wearable device, another apparatus, a combination thereof, and/or the like. A mobile device may comprise, for example, a phone, a tablet, a notebook, and combination thereof, and/or the like.

According to one embodiment, at least one measurement recorded with the at least one sensor **3** may be communicated through the communication means according to a clock, at intervals required by a processing unit, at intervals required by the communication means, at intervals specified by a remote device, at intervals selected by the user, a combination thereof, and/or the like. The sensor measurements may comprise, for example, the temperature measurements of the at least one liquid, gel and/or gas(es) in the fluidic circuit. According to one embodiment where the at least one sensor **3** is a temperature sensor, said temperature sensor may, for example, communicate temperature measurements to the at least one processing unit. According to one embodiment, the temperature measurements may comprise differential temperature measurements, relative temperature measurements, absolute temperature measurements, a combination thereof, and/or the like. The absolute temperature measurements may comprise degrees Celsius, degrees Fahrenheit, a combination thereof, and/or the like.

According to one embodiment, the sleep device further comprises a fluidic circuit permitting fluid communication between the thermal pad **2** and the thermal unit.

According to one embodiment, the thermal unit **4** includes one or more temperature regulators for regulating the temperature of the thermal transfer fluid to be circulated to the fluidic circuit and into the thermal pad **2.** Any appropriate temperature regulator, for example, a heater, a cooler, or both, may be used. For example, the thermal unit may comprise thermal electric modules, such as Peltier devices, to cool or heat a thermal transfer fluid which is pumped through input transfer tube **5a** to the thermal pad **2.** Other cooling or heating means such as a refrigeration compressor could also be used instead or in addition to thermal electric modules. Other cooling or heating means may use chemical reactions. For example, resistive heaters, fans, or other elements may also or alternatively be used. Other components of the thermal unit may include one or more heat exchangers, heat sinks, thermal electric modules, a pump, a valve, fan, electronic control circuits, software, user interface, thermal transfer fluid reservoir, unit enclosure, connections for incoming electrical power, and/or thermal transfer fluid connections for the thermal applicator.

The components may be assembled such that the heat sink(s) are placed in thermal contact with one side of the thermal electric modules and a heat exchanger is placed in thermal contact with the opposite side of the thermal electric modules away from the heat sink. The heat exchanger can be constructed from any known material and design for the purpose. One example of which is a copper tube imbedded within an aluminum plate. A fan may be used to remove heat from the heat sink. Portions of the assembly can be insulated to reduce parasitic heat loads on the heat exchanger.

According to one embodiment, at least one metal wire, a at least one metal sheet, and/or a at least one metal rod may be configured as a heat sink. The heat sink may be connected to at least one additional thermal element. The at least one metal wire may, for example, be configured as a woven wire mesh. The woven wire mesh may comprise a highly thermally conductive material. The woven wire mesh may, for example, be woven into at least one material suspended from a support structure. Alternatively, the woven wire mesh may, for example, be a distinct layer from the at least one material suspended from a support structure. Distinct layers may be separated by at least one material. Furthermore, the woven wire mesh may, for example, be a distinct layer from the woven fiber mesh of a capacitive proximity sensor.

According to one embodiment, a temperature sensor or thermocouple may, for example, be employed to monitor the temperature of the thermal transfer fluid in the at least one fluidic circuit. The activation of a thermal unit may, for example, comprise adjusting a flow rate of at least one liquid, gel, air, and/or gas.

According to one embodiment, the thermal unit uses a pump to make the thermal transfer fluid circulate through the heat exchanger and the fluidic circuit. The pump can be of any appropriate type, such as for example centrifugal, piston, gear, diaphragm etc. A thermal transfer fluid reservoir is incorporated to provide additional thermal transfer fluid to replenish the thermal transfer fluid lost for any reason. For example, the reservoir may be configured as a cartridge that is removable (or not removable) from the thermal unit **4.** Thus, a reservoir can be an integral fillable component within the thermal unit or can be constructed as a replaceable and/or disposable cartridge.

According to one embodiment, the sleep device **1** comprises a power source. By way of example and not limitation, the power source may comprise at least one of the following: an electric outlet providing alternating current, at least one battery providing direct current, a solar panel configured to charge at least one battery, a generator providing at least one of alternating and direct current, a combination thereof, and/or the like. According to one embodiment, the sleep device **1** further comprises a power switch. The power switch may, for example, be configured to provide at least one of the following modes: power on/off, heating only, cooling only, thermal regulation according to the user temperature profile **PROF,** a combination thereof, and/or the like. The thermal regulation mode may, for example, comprise heating and cooling functions. The sleep device may, for example, further comprise a converter of at least one of voltage and current.

According to one embodiments, the thermal unit and comprised equipment such as at least one fan, compressor, pump, power source, thermostat etc., are associated with at least one sound reducing container. The sound reducing container may, for example, comprise at least one of the following sound reducing materials: at least one sound reducing mat, at least one sound reducing panel, sound reducing insulation, at least one liquid configured to attenuate sound, a combination thereof, and/or the like. Associated equipment, for example, may be located remotely from other elements of the sleep device. Remote locations may, for example, comprise one or more separate containers or installations enclosed in one or more walls and/or the like. According to one embodiment, the housing 4 comprises the at least one sound reducing container.

### METHOD

According to one embodiment, the predefined data **DATA1** are defined by the user, preferably before a first use of the sleep device. According to one embodiment, the predefined data **DATA1** comprises at least one user identifier such as the user name. The predefined data **DATA1** may further comprise demographic information concerning the user (including but not limited to age, gender, race and/or the like) and/or the user lifestyle (such as for example diet, body mass index, smoking or not, sporty or not, utilization of energetic drinks or substances such as coffee etc., typical sleeping position and/or the like).

According to one embodiment, the predefined user data **DATA1** comprises information obtained from a sleep assessment survey. The sleep assessment survey may comprise information concerning user sleep such as, for example, the duration of an average sleep cycle of the user, the time at which the user goes to sleep or wakes up during the week days and/or the weekend, the user overall perceived quality of sleep, the user sleep habits, the user sensitivity to the cold and/or the like. Said predefined data **DATA1** may be modified by the user after the first use of the sleep device.

According to one embodiment, the dynamic user data **DATA2** received by the method comprises data collected from at least one of the sleep device sensors. According to a preferred embodiment, the dynamic user data **DATA2** received by the method comprises data collected from a user interaction with at least one sensor of the sleep device. According to this embodiment, the dynamic user data **DATA2** comprises kinematic and kinetical measurements of the user movements during the sleep cycle, ambient and/or temperature measurements, noise such as snorting measurement and/or the like.

According to one embodiment, the dynamic user data **DATA2** comprises a previous user temperature profile generated by a previous use of the method of the present invention.

According to one embodiment, the method further comprises a preprocessing step where the dynamic user data **DATA2** received from the at least one sensor of the sleep device are for example processed to perform noise reduction, remove or reject artefact and/or the like.

According to one embodiment, the method of the present invention comprises the step of calculating at least one sleep evaluation variable **VAR** using at least one part of the dynamic user data **DATA2** and/or predefined user data **DATA1.** According to one embodiment, at least one sleep evaluation variable **VAR** is obtained from the confrontation of a sleep parameter extrapolated from the dynamic user data **DATA2** collected from at least one of the sleep device sensor to a reference value. According to one embodiment, said reference value is calculated as the average value obtained for said sleep parameter over multiple night of sleep device utilization. According to an alternative embodiment, the reference value is obtained from literature or is calculated as the sleep parameter averaged on a representative population, wherein the subjects comprised in the population have at least one characteristic in common with the user such as the gender, age, geographic localization and/or the like. For example, the sleep parameter may be the cumulation of time lapses during which the motion sensor has recorded a user movement during the sleep cycle of the user, the gradient of temperature registered by a body temperature sensor, a period of time during which the user in a rapid eye movement sleep, for example, obtained from the analysis of the electromyographic or electroencephalographic signal and/or the like. According to one embodiment, the confrontation between the sleep parameter and the reference value is done by thresholding.

According to one embodiment, at least one sleep evaluation variable **VAR** is extrapolated from the predefined user data **DATA1.** According to this embodiment, the at least one sleep evaluation variable **VAR** may be the average duration of a sleeping cycle of the user, for example, during the week days or during the weekend.

According to one embodiment, the at least one sleep evaluation variable **VAR** is calculated using a scoring model **MODs** and at least one part of the dynamic user data **DATA2** and/or predefined user data **DATA1.** According to one embodiment, the scoring model **MODs** is based on a machine learning model wherein the training phase may be an unsupervised training or a supervised training. According to the embodiment where the machine learning method implements a supervised machine learning, the method has access to a database comprising set of training data. The scoring model **MODs** may be a machine learning model such as, but not limited to multiple linear regression model, Bayesian network or recurrent neural network. The scoring model **MODs** may be constructed for a subset of relevant features selected using a feature selection technic.

According to one embodiment, the scoring model **MODs** is constructed to calculate a score evaluating the user quality of sleep.

The method of the present invention further comprises the step of generating **GEN** a user temperature profile **PROF** using a profiling model **MODp** receiving as input the at least one sleep evaluation variable **VAR** and at least one part of the dynamic user data **DATA2** and/or predefined user data **DATA1.**

The user temperature profile **PROF** generated from the profiling model **MODp** comprises a plurality of associations, each of said association comprising a temperature and a period of time of the user sleep cycle.

According to one embodiment, the profiling model **MODp** is configured to further receive as input a first timestamp corresponding to the begin of the cycle and a second timestamp corresponding to the end of the cycle. According to one embodiment, the first timestamp corresponding to the begin of the sleep cycle and the second timestamp corresponding to the end of the sleep cycle are derived from the processing of the dynamic user data **DATA2.** Said first and second timestamp may be obtained, for example, from the beginning and end of the time period during which the user has interact with movement sensor or any other sensors of the sleep device.

According to one embodiment, the profiling model **MODp** is a machine learning model. According to one embodiment, the profiling model **MODp** is based on a machine learning model wherein the training phase may be an unsupervised training or a supervised training. According to the embodiment where the machine learning method implements a supervised machine learning, the method has access to a database comprising set of training data. The profiling model **MODp** is a machine learning model such as but not limited to mistake bound model or a reinforcement learning model (including value function approaches, Monte Carlo methods, temporal difference method etc.).

According to one embodiment, the profiling model **MODp** further receives as input a temperature profile **PROF** generated by a previous use of the method according to the embodiments of the present invention.

According to one embodiment, at a first use of the sleep device a preliminary user temperature profile is obtained using a classifier receiving as input the predefined user data **DATA1** and configured to classify the user according to the predefined user data **DATA1** into a user category associated to one predefined user temperature profile. The classifier may be, for example, a linear classifier (e.g. support vector machine, linear discriminant analysis, etc.), a quadratic classifier, a k-nearest neighbor and the like.

The method of the present invention further comprises the step consisting in regulating **REG** the sleep device by generating a temperature instruction calculated with the user temperature profile **PROF.**

According to one embodiment, the method of the present invention further comprises the step of transmitting the temperature instruction to the sleep device.

### SYSTEM

A system comprising a sleep device including at least one sensor, a thermal control unit, executing the temperature instruction obtained from the method described in the embodiments hereabove and a user interface for user interaction.

According to one embodiment, the thermal control unit is comprised in the housing **4** of the sleep device.

According to one embodiment, the thermal control unit is configured to control the thermal unit in order to adjust the temperature of the thermal pad **2** during a sleep cycle of the user according to the user temperature profile **PROF** generated accordingly to the method described hereabove.

According to one embodiment, the thermal control unit comprises a processing unit. The at least one processing unit may, for example, comprise a computing device, a smart device, a microcontroller, a microprocessor system, a combination thereof, and/or the like. A processing unit may include hardware configured to execute the instructions of a computer program by performing the basic arithmetical, logical, and input/output operations within a system. In one embodiment, a processing unit may comprise a central processing unit (CPU) with associated hardware (e.g. power, input/output, data bus interface, display, etc.). In one embodiment, a processing unit may comprise a microcontroller configured to control the thermal unit in order to execute the temperature instruction obtained from the method described hereabove.

According to one embodiment, the system includes at least one user interface. A user interface may comprise components and/or systems employed to effectuate human and machine interactions. The interaction communicates operation and control desires of a user, and/or feedback from a machine. The user interface may comprise a graphical user interface, at least one switch, at least one indicator, at least one display, at least one touch screen, a combination thereof, and/or the like. The user interface may, for example, be configured via an external device or a remote device. The user interface may also, for example, be configured to receive at least one of the following: predefined user data **DATA1** associated with at least one user, preference information associated with at least one user, a combination thereof, and/or the like. Information sent to and/or from the display and/or user interface may be encrypted.

According to one embodiment, the system may comprise multiple sleep devices each associated to a thermal unit. According to this embodiment, the thermal pad of the different sleep device may be mechanically connected but not in fluid communication within each other. The system comprising this combination of multiple pad each separately regulated by a different thermal unit may be configured to apply different temperature to different body parts at the same time.

According to one embodiment, the system of the present invention is configured to communicate with other systems or devices from which collect or transfer information. Said systems or devices, may be monitoring sleep devices such as a smart bracelets monitoring a subject movements and/or position, a sound machine to record or diffuse sounds, lighting devices and/or the like.

While various embodiments have been described and illustrated, the detailed description is not to be construed as being limited hereto. Various modifications can be made to the embodiments by those skilled in the art without departing from the true spirit and scope of the disclosure as defined by the claims.

### BRIEF DESCRIPTION OF THE DRAWINGS

**Figure 1** is a schematic block diagram of the method for regulation of a sleep device in which the present invention may be embodied.
**Figure 2** is a schematic representation of the sleep device according to one embodiment.
**Figure 3** is a schematic representation of the sleep device according to the embodiment comprising an input transfer tube **5a** and an output tube transfer **5b** putting in fluidic communication the input and output of the thermal pad **2** with the thermal unit.
**Figures 4** to **6** report different patterns of the hollow conduits **21** of the thermal pad **2.**

### REFERENCES

DATA1 - predefined user data;
DATA2 - dynamic user data;
DEF - step of defining a predefined user data;
RCV - step of receiving dynamic user data;
K - step of calculating at least one sleep evaluation variable;
VAR - at least one sleep evaluation variable;
GEN - step of generating a user temperature profile;
PROF - user temperature profile;
REG - step of regulating the sleep device;
MODs - scoring model;
MODp - profiling model;
1 - sleep device;
2 - thermal pad;
3 - at least one sensor;
4 - housing;
5a-5b - input transfer tube and an output tube transfer;
21 - hollow conduits.

## Claims

1. Method for regulating the temperature of a sleep device, comprising at least one sensor, the method comprising the following steps:
a. defining (DEF) a predefined user data (DATA1);
b. receiving (RCV) dynamic user data (DATA2), said dynamic user data comprising data collected from a user interaction with at least one sensor of the sleep device;
c. calculating (K) at least one sleep evaluation variable (VAR) evaluating the quality of sleep of the user using at least one part of the dynamic user data (DATA2) and/or predefined user data (DATA1);
d. generating (GEN) a user temperature profile (PROF) using a profiling model (MODp) having as input the at least one sleep evaluation variable (VAR) and at least one part of the user data (DATA1, DATA2); where the user temperature profile (PROF) comprises a plurality of associations, each of said association comprising a temperature and a period of time of the user sleep cycle; and
e. regulating (REG) the sleep device by generating a temperature instruction calculated with the user temperature profile (PROF).

2. The method according to claim **1,** wherein the at least one sleep evaluation variable (VAR) is calculated using a scoring model (MODs) and at least one part of the dynamic user data (DATA2) and/or predefined user data (DATA1).

3. The method according to anyone of claim **1** or **2,** wherein the dynamic user data (DATA2) comprises a previous user temperature profile generated by a previous use of the method.

4. The method according to anyone of claims **1** to **3,** wherein the predefined user data (DATA1) comprises information obtained from a sleep assessment survey.

5. The method according to anyone of claims **1** to **4,** further comprising a step of preprocessing of the dynamic user data (DATA2) received from the at least one sensor of the sleep device.

6. The method according to anyone of claims **1** to **5,** wherein the profiling model (MODp) is configured to further receive as input a first timestamp corresponding to the begin of the sleep cycle and a second timestamp corresponding to the end of the sleep cycle.

7. The method according to claim **6,** wherein a first timestamp corresponding to the begin of the sleep cycle and a second timestamp corresponding to the end of the sleep cycle are derived from the processing of the dynamic user data (DATA2).

8. The method according to anyone of claims **1** to **7,** further comprises the step of transmitting the temperature instruction to the sleep device.

9. A sleep device using body temperature conditioning, comprising:
a. a thermal pad;
b. at least one sensor;
c. a communication means configured to transmit the information detected by the at least one sensor and receive the user temperature profile (PROF) generated by the method according to anyone of claims **1** to **8**; and
d. a thermal unit adjusting a temperature of the thermal pad during a sleep cycle of the user according to the user temperature profile (PROF) generated.

10. The sleep device according to claim **9,** wherein at least one of the at least one sensor is at least one of the following: an ambient temperature sensor, a movement sensor, a humidity sensor, a sound sensor or/and a light sensor.

11. A system comprising a sleep device according to anyone of claim **9** or **10,** a user interface for user interaction and a thermal control unit, executing the temperature instruction obtained from the method according to anyone of claims **1** to **8.**
